# EUROPEAN PATENT APPLICATION

(11) **EP 3 936 609 A1**
(43) Date of publication of application: **12.01.2022**
(21) Application number: 21190079.0
(22) Date of filing: 14.08.2015
(51) Int. Cl.: C12N 5/073, C12N 5/0775, A61P 29/00, A61P 37/02

(54) **STEM CELL COMPOSITIONS AND METHODS OF PRODUCING STEM CELLS FOR THERAPEUTIC APPLICATIONS**

(30) Priority: 14.08.2014 US 201462037600 P
(62) Divisional of application: 15757006.0
(71) Applicant: Avita International Ltd., Road Town, Tortola (VG); Fundação Butantan, São Paulo, SP, CEP 05503-900 (BR)
(72) Inventor: KERKIS, Irina, Sao Paulo (BR); GLOZMAN, Sabina, Naharya (IL)
(74) Representative: Barker Brettell LLP

(57) **Abstract**

The present method relates to methods of expanding or increasing stem cell production obtained from donor samples. The methods preferably including the steps of harvesting cells from minimally manipulated tissue using multiply harvesting cycles to increase the number of obtained stem cells.

## Description

### BACKGROUND

Stem cells are an attractive source of cells for therapeutic applications, medical research, pharmaceutical testing, and the like. Unfortunately, the use of the cells as an allogeneic graft is problematic. The expansion of stem cells to large quantities is a pre-requisite for cell therapy, but adult MSCs have limited proliferative capacity. It is of common knowledge in the state of art that principal method used for MSCs from bone marrow (BM) isolation is their capacity to adhere onto plastic, when whole bone marrow was placed in plastic culture dishes and after 4 hours the non-adherent cells were washed out. This protocol can also include the density centrifugation of BM in solutions of high density with low viscosity and low osmotic pressure (eg. Ficoll, Percoll) to obtain the mononucleated fraction of BM which contains MSCs. Additionally MSCs from solid tissues like bone could be isolated by placing a small piece of bone inside the flask and cells grow out of bone explants. Alternatively, the collagenase digestion process can also be used. However, contamination of the cells with other types of cells like haematopoietic stem cells results in a heterogeneous population likely occurs, compromising the purity of the MSCs sample. In order to overcome this problem, two similar techniques, magnetic bead sorting and fluorescence-activated cell sorting (FACS), have been developed. Otherwise, the fluorescence-activated cell sorting (FACS) is a method to isolate MSCs. The ex-vivo expansion is next essential step after MSCs isolation, which allows the free ex vivo auto renovation of MSCs targeting production of therapeutically significant cell number, which are depends on several factors, such as donor dependent (age, sex, the presence of trauma, and the presence of systemic disease) or method dependent. Even

MSCs can be expanded tremendously within a relatively short period of time due to rapid proliferation, however no methods exist in the state of art which allows to produce MSCs in therapeutically relevant number without extensive *in vitro* cultivation, which means multiple rounds of cells passing from flask to flask using enzymatic treatment. MSCs expansion under such conditions has shown to gradually reduce them maximal differentiation potential of MSCs. Extensive subcultivation impairs the cells' function resulting in cellular senescence that is associated with growth arrest and apoptosis. On the other hand, there are data suggesting that prolonged culture could result in spontaneous transformation acquiring tumorigenic potential. In addition, it was shown that particular properties of MSCs are lost during culture. The cardioprotective effect of MSCs is reduced in cells at passage above 5 and 10 compared to those below of this passage. This could be explained by the reduced vascular endothelial growth factor release potential (Crisostomo et al., 2006; Digirolamo et al., 1999; Muraglia et al.,2000; Rubio et al., 2005; Stenderup et al., 2003). Therefore, all methods present in state of art should be made compromise between the yield and the quality of the expanded cells, which limited their use in cells therapy.

Method and cell composition described in present invention overcome the aforementioned limitations, such as, multiple cell passages, cellular senescence, reduced differentiation and reduced of growth factors production, limited number of cryopreserved cells. In contrast we proposed reduced cell passages, reduced in vitro cell manipulation and in parallel increase of differentiation capacities and growth factors production, stable karyotype, practically unlimited number of cryopreserved cells.

All publications, patents, and patent publications cited are incorporated by reference herein in their entirety for all purposes.

### SUMMARY OF THE INVENTION

The present invention is directed methods of obtaining and/or expanding in vitro a stem cell population isolated from explant tissue. The method preferably comprises the step of culturing the tissue in culture medium for at least one harvest cycle. The harvest cycle generally comprises the steps of maintaining the tissue in culture medium to explant cells for a period of time sufficient to obtain semi-confluent stem cell culture and collecting these cells for the final stem cell population. The tissue is preferably minimally manipulated prior to each harvest cycle.

In a particular embodiment, the present invention is directed to an *in vitro* method of obtaining from an explant tissue an expanded population of primary derived stem cells, the method comprising:
a. washing and minimally manipulating the explant tissue;
b. culturing the explant tissue in vitro in a first culture surface with culture medium to establish a culture of primary derived stem cells;
c. collecting primary derived stem cells from the culture medium; and
d. transferring the explant tissue to second culture surface with culture medium;
e. repeating steps b., c. , and d. at least once with the explant tissue, thereby obtaining an expanded population of primary derived from the explant tissue.

These methods preferably comprise washing and minimally manipulating the explant tissue. Preferably, the explant tissue is not enzymatically or chemically treated or dissected prior to each harvest cycle. The method of obtaining an expanded population of primary derived stem cells from an explant tissue preferably also comprises the step of passaging the primary derived stem cells on growth medium and collecting stem cells from the growth medium. Passaging may be done after each harvest cycle multiple times or at the end.

The methods may further comprise passaging the explanted cells and culturing the passaged cells in a growth medium. In some aspects, culturing the tissue in culture medium for at least one harvest cycle comprises culturing the tissue initially in culture medium containing less than 5% serum and then culturing the tissue in culture medium containing 15% serum. The duration for culturing the tissue in culture medium containing less than 5% serum is three days. The duration for culturing tissue in culture medium containing 15% serum is seven days. In some implementations, the culture medium and the growth medium is DMEM, for example, the culture medium is DMEM-F12 and/or the growth medium is DMEM-low glucose. In some aspects, the growth medium is not supplemented with non-essential amino acids. In some embodiments, the explanted cells and/or passaged cells have a stable karyotype.

The present invention is also directed to therapeutic compositions of stem cells comprising explanted cells obtained from at least the first harvest cycle. In some embodiment, the composition comprises explanted cells obtained from the first three harvest cycles, the first 65 harvest cycles, or the first 100 harvest cycles. In some aspects, the composition comprises passaged cells obtained from at least passage 1 (PI), from P1 to P3, or from P1 to P50. In some implementations, the composition may be stored in frozen condition without altering its expression of stem cell markers after the composition is thawed.

In some embodiments of the methods and compositions of the present invention, the stem cell is of neuronal crest or peripheral neuronal system origin. In some aspects, the stem cells are adult or postnatal stem cells, non-embryonic stem cells. In some embodiments, the tissue is dental pulp or postpartum tissue, for example, umbilical cord or placenta.

Aspects or embodiments of the invention may also be provided according to the following paragraphs:
1. A method of expanding a primary derived stem cell population *in vitro* from an explant tissue, the method comprising culturing the explant tissue in a culture medium for at least two harvest cycles, wherein a harvest cycle comprises maintaining the tissue in culture medium for a period of time sufficient to obtain a semi-confluent stem cell culture and collecting the semi-confluent stem cells for the final stem cell population and the explant tissue is minimally manipulated prior to each harvest cycle.
2. An in vitro method of obtaining from an explant tissue an expanded population of primary derived stem cells, the method comprising:
   a. washing and minimally manipulating the explant tissue;
   b. culturing the explant tissue in vitro in a first culture surface with culture medium to establish a culture of primary derived stem cells;
   c. collecting primary derived stem cells from the culture medium; and
   d. transferring the explant tissue to second culture surface with culture medium;
   e. repeating steps b., c. , and d. at least once with the explant tissue, thereby obtaining an expanded population of primary derived stem cells from the explant tissue.
3. The method of paragraph 1 or 2, further comprising the step of passaging the primary derived stem cells on growth medium and collecting stem cells from the growth medium
4. The method of any one of the preceding paragraphs, wherein culturing the tissue in culture medium for at least one harvest cycle comprises culturing the tissue initially in culture medium containing less than 5% serum and then culturing the tissue in culture medium containing 15% serum.
5. The method of paragraph 4, wherein the duration for culturing the tissue in culture medium containing less than 5% serum is three days.
6. The method of paragraph 5, wherein the duration for culturing tissue in culture medium containing 15% serum is seven days.
7. The method of any one of the preceding paragraphs, wherein the culture medium and the growth medium is DMEM.
8. The method of paragraph 7, wherein the culture medium is DMEM-F12.
9. The method of paragraph 7, wherein the growth medium is DMEM-low glucose.
10. The method of paragraph 9, wherein the growth medium is not supplemented with non-essential amino acids.
11. The method of any one of the preceding paragraphs, wherein the explanted cells and/or passaged cells have a stable karyotype.
12. A therapeutic composition of stem cells comprising stem cells obtained from the method of paragraph 1 or paragraph 2.
13. The therapeutic composition of paragraph 12, wherein the composition comprises explanted cells obtained from the first three harvest cycles.
14. The therapeutic composition of paragraph 12, wherein the composition comprises explanted cells obtained from the first 65 harvest cycles.
15. The therapeutic composition of paragraph 12, wherein the composition comprises explanted cells obtained from the first 100 harvest cycles.
16. The therapeutic composition of any one of paragraphs 12-16, further comprising passaged cells obtained from at least passage 1 (P1).
17. The therapeutic composition of any one of paragraphs 12-16, wherein the composition comprises passaged cells obtained from P1 to P3.
18. The therapeutic composition of any one of paragraphs 12-16, wherein the composition comprises passaged cells obtained from P1 to P50.
19. The therapeutic composition of any one of paragraphs 12-16, wherein the composition may be stored in frozen condition without altering its expression of stem cell markers after the composition is thawed.
20. The method of any one of paragraphs 1-11 or the therapeutic composition of any one of paragraphs 12-19, wherein the stem cell is of neuronal crest or peripheral neuronal system origin.
21. The method of any one of paragraphs 1-11 or the therapeutic composition of any one of paragraphs 12-19, wherein the tissue is dental pulp.
22. The method of any one of paragraphs 1-11 or the therapeutic composition of any one of paragraphs 12-19, wherein the stem cells are adult or postnatal stem cells, non-embryonic stem cells.
23. The method of any one of paragraphs 1-11 or the therapeutic composition of any one of paragraphs 12-19, wherein the tissue is postpartum tissue.
24. The method of any one of paragraphs 1-11 or the therapeutic composition of any one of paragraphs 12-19, wherein the postpartum tissue is selected from the group consisting of: umbilical cord and placenta.
25. The method of paragraph 3, wherein the primary derived and passaged stem cells are mixed to form a batch of primary derived and passaged stem cells from a single explant tissue.
26. The method of paragraph 2, wherein step e is repeated at least 30 times.
27. The method of any of the proceeding paragraphs wherein the explant tissue is not enzymatically or chemically treated or dissected prior to each harvest cycle.

### BRIEF DESCRIPTION OF THE DRAWINGS

FIG. 1 depicts a schematic for the isolation of stem cells from the umbilical cord (UC). Panel A depicts the isolation of a 5 cm piece of UC. Panel B depicts washing of the UC tissue; Panel C depicts the UC tissue cut into small pieces. Panel D represents the small pieces of UC tissue that has been mechanically transferred into basal medium and cultured in a plastic culture plate. Panel E shows the adherence of stem cell positive for CD105 on the culture surface. Panel F depicts the culture of mesenchymal stem cells.
FIG. 2 depicts methods of culturing stem cells on an industrial scale.
FIG. 3 depicts the use of non-enzymatic method of stem cells isolation and mechanical transfer in biotechnology, medicine, pharmaceuticals, cosmetology, nutrition etc.
FIG. 4 depicts the isolation of the fragments of different donors and co-culture of these fragments in the same flask. Fragments obtained from different HLA-matched, partially matched, and/or unmatched donors may be cultured in the same flask for the production of MSC so long as they do not present any signal of immune reaction.
FIG. 5 depicts conventional method of tissue-source cryopreservation for "one time" stem cells isolation (a) and the tissue-source cryopreservation of the present application (b).
FIG. 6 depicts the principle differences between normal MSCs and EP or iPS cells.
FIG. 7 depicts the growth of hIDPSC in two types of growth media.
FIG. 8 depicts the effect of IDPSCs intraperitoneal inoculation on cytokine production by lung cells of C57Bl/6 mice infected with *Mtb* and *M*. *bovis* strains.

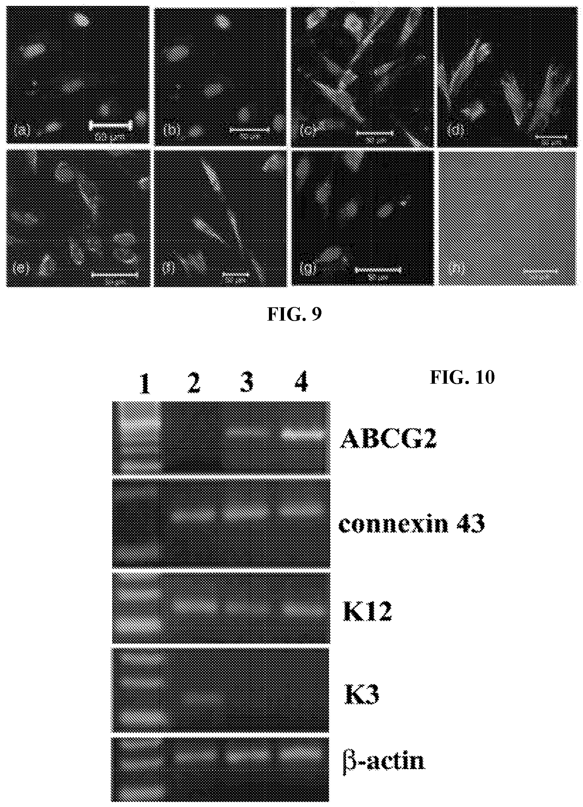

FIG. 9 depicts immunostaining of limbal stem cell markers in IDPSCs for corneal transplantation visualized through fluorescent microscopy. The scale bar represents a distance of 50 µm. Panel A depicts nuclear localization of p63 in IDPSC. Panel B is a merged image of panel A with the DAPI nuclei stain. Panel C depicts membrane localization of integrin β1. Panel D depicts detection of vimentin, an intermediate filament. Panel E depicts detection of connexin 43, which a cell membrane protein. Panel F depicts detection of ABCG2, a plasma membrane protein. Panel G depicts weak positive immunostaining of K3/12. Panel H depicts the negative control.
FIG. 11 depicts niches of stem cells in dental pulp. Panel A that a tooth is composed of a crown section and root section. Panel B shows the location of the perivascular niche (a) and nerve plexus (b) for dental pulp. Panel C depicts immunostaining of nestin in the perivascular niche (a) and the nerve plexus (b). Panel D shows the location of the subodontoblastic niche (c) and the cell-free and cell-rich zones (d). Panels E an F depicts immunostaining of nestin in the subodontoblastic niche (c) and the cell-free and cell-rich zones (d), respectively.
FIG. 12 depicts putative niches of nerve stem cells in umbilical cord. Panel A shows the expression of neurofilaments (NF) in the subamniotic epithelium. Panel B shows the expression of low affinity nerve growth factor (p75 or CD271) in the vein. Panel C shows the positive immunostaining of beta-III tubulin of stem cells isolated from putative nerve stem cell niches. Panel D shows positive immunostaining of GFAP of stem cells isolated from putative nerve stem cell niches.
FIG. 13 depicts the expression of transcription factors such as Oct 3/4, nanog, and Sox2 in different stem cell niches in umbilical cord tissue. White arrears depict cells with expressing these transcription factors while black arrows depict cells with that lack expression of these transcription factors.

### DETAILED DESCRIPTION OF THE INVENTION

US Patent Application Publication No. 2004/0136967 discloses a method for obtaining stem cells from an umbilical cord matrix comprising isolation of cells from explants of the umbilical cord or freeing cells enzymatically e.g., collagenase or trypsin. The tissue sections are then covered with another glass slide and cultured in a complete medium. Thus the prior art does not teach repeatable use of explant cultures.

Unlike the methods of scaling up stem cell populations of the prior art, the present invention uses multiple harvesting cycles to increase the population of cultured stem cells. Also unlike the methods of prior art, the origin tissue is not dissected but is mechanically sliced or grinded for repeated harvest cycles. The tissue should be minimally manipulated. A harvest cycle refers to the cycle of transferring the tissue to a fresh culture surface to establish a new explant culture of, for example, stem cells. In the present invention, the cycles of harvesting (tissue transfer for stem cell sourcing) may be repeated through multiple cycles, for example from 1-100 transfers. The multiple harvest cycles establish multiple primary cultures to provide a large-scale supply of stem cells with unique features. In other words, the present invention provide a method of robust culturing stem cells in large scale by repetitive primary culturing of stem cells harvested from the minimally manipulated tissue source through repeated harvesting cycles of the same tissue. Unexpectedly, repeating the harvest cycle results in increased number of adhered cells obtained in each cycle. And surprisingly, we have found that by repeated explant culturing, through non-enzymatic harvesting of minimally manipulated tissue pieces exposed to culture growth medium containing antibiotic/anti-mycotic and supplements, we can initiate passage 0 (P0) conditions multiple times, which improve chances for safety due to karyotype stability at low passages during providing sufficient stem cell numbers through repeated harvest cycles.

The method of minimally manipulated tissue was known partially in prior art for non-therapy applications, mainly diagnostic purposes. In particular, these purposes include cultivation of organ (e.g. cornea) and tissue explant for evaluation of the effects of toxic, carcinogenic, therapeutic substances, biological agents or susceptibility testing. Recently we have disclosed the utilization of this technique for the isolation of dental pulp stem cells. The main objective of this approach is to maintain cultured tissue architecture, for example, the parenchyma and stroma are conserved in terms of its structure and function. So the organ *in vitro* during culturing resemble the organ functions *in vivo.* In addition we also utilized the approach to culture the explant is used for isolation of the cells from a piece of organ that previously was used in the state of the art for diagnostic, screening methods: the isolated tissue is isolated, minimally treated under aseptic conditions, chopped to small pieces and placed in cell culture dish containing culture medium.

Explant culture is a culture where the cells are left in their surrounding extracellular matrix that more accurately mimic the *in vivo* stem cells/progenitors migration from the niches to external environment, and progenitor cells migrate out of the tissue and adhere to the surface of the culture dish. Both approaches have similarities, since they preserve the environment and stem cell niches. Utilizing this method for stem cell expansion is beneficial due to the tissue under the cultivation time is subjected to hypoxia. However, the culture media provides enough supply of nutrients to support the explant cells of the minimally manipulated tissue and cells within the tissue to be maintained in a viable stage. This benefit is unexpected compared to the prior art teachings, and provide a great advantage in maintaining the undifferentiated state of stem cells. Therefore the mechanical transfer of the dental pulp, which is a small organ, and explant, is a strategy to continuously isolate stem cells in order to maintain their primordial characteristics unchanged after isolation. The dental pulp is a loose connective tissue and this organ can vary in weight. DP, which is lighter, tends to flow without adhesion and cell outgrowth. To accelerate adherence of pulp tissue to the substrate sterile coverslips can be used even providing more hypoxic conditions. Use of coverslips is another example to induce hypoxia within tissue stem cell niche during multiple harvesting cycles.

In one embodiment, the present invention is directed to methods of expanding in vitro through at least one harvesting cycles from minimally manipulated tissue to an expanded population of primary derived and passaged stem cells. During each harvest cycle, the number of explant cells are at least doubled the number of cells isolated with classical enzymatically derived stem cell expansion. The number of harvest cycles may be at least three cycles (H1-H3), at least 65 cycles (H1-H65) or up to 100 harvest cycles (H1-H100). Each harvest cycle comprises maintaining the minimally manipulated tissue fragment (e.g. non-enzymatically or chemically treated, not dissected) in supplemented culture medium with antibiotics to explant cells for a period of time sufficient to obtain semi-confluent stem cell culture and collecting these cells for the final stem cell population. This generation of cultured cells is the primary passage (P0) of a harvest cycle

The methods comprise washing the minimally manipulated tissue and maintaining the tissue *in vitro* through at least one harvest so that stem cells, such as various types of mesenchymal multipotent and pluripotent stem cells, attach to the culture surface or beads. Each explant culture from a harvest cycle is P0 and serves a source for sub-sequential passaging or bioreactor system. The explant culture may be passaged for up to 50 passages. Cells may be passaged enzymatically or non-enzymatically. During repeated harvest cycles, the tissue and cells may be maintained in growth factors and/or DMEM supplemented growth medium containing antimicrobial solution. In some aspects, the total number of stem cells obtained through a piece of tissue using the methods of the invention is at least triple the number of stem cells that could be obtained through classical enzymatically derived stem cell expansion. In some implementations, the total number of stem cells obtained through the methods of the invention is at least 3 to at least 65 folds higher than the number of stem cells obtained through classical enzymatically derived stem cell expansion. Accordingly, the present invention is directed to methods of increasing stem cell number obtained from single or mixed donor's tissue/fluid source in comparison with classic method with same tissue source.

The tissue may be cryopreserved prior to establishing explant cultures. Adhered cells from explant cultured and subsequent passages may also be cryopreserved. Cell viability after freezing is greater that 50-90%, which makes them suitable for industrial and therapeutic applications. In some aspects of the method, the therapeutic composition obtained from the batch that mix frozen-thawed mesenchymal stem cells obtained during multiple harvesting cycles according the described methods. The batch may comprise mixed passages of cells from a mixture of harvest cycles or the same harvest cycle. A batch of cells for research and clinical applications may be a composition comprising P0 cells from different harvest cycles.

In another embodiment, the present invention is directed to methods of cell therapy comprising injecting and/or implanting a batch of isolated according to the methods of invention into a human or animal at a location where treatment is required. The batch of cells may be mixed cells obtained from different harvest cycles and passages directly frozen stocks. The batch of cells may also be cultured from frozen stocks prior to administration into the human or animal. In some implementations, the batch of cells is tested for functional parameters required for targeted therapy, such as relevant factors released and/or relevant biomarkers tested. The final stem cell population isolated according to the methods of invention expresses mesenchymal markers. The final stem cell population may also express pluripotent markers, neuroectodermal markers, and/or pericytes markers. In some embodiments, the final stem cell population preferably expresses mesenchymal and some pluripotent markers, especially neuroectodermal, epithelial markers, and/or pericytes markers. In some embodiments, the stem cells preferably lack of MHC class II expression, such as HLA II (HLA-DR) expression. For neuronal applications, preferable CNS factors, markers tested, such as beta tubulin, SOX2; or SOX10; or neuronal growth factors and peptides, such as GDNF, NGF, and BDNF. The batch of cells may have its differentiation capacity tested. In some aspects, the invention is also directed to buffered therapeutic composition for cell therapy wherein the pH is not acidic and toxic for cells.

An embodiment of the present invention is also directed to methods of obtaining primary stem cells in expanded culture *in vitro* from minimally manipulated tissue, wherein the tissue is not treated or dissected full disintegration by chemicals or enzymes. These methods comprise: continuously, periodically, or intermittently harvesting adherent stem cells from said tissue in cell culture chamber maintained in supplemented growth medium containing anti-microbial solution; and passaging the cultured cells from first harvest repeating steps. In preferred embodiments, the harvesting step is repeated and the passaging step is repeated at least one, two, or three times for each harvesting step. The resulting stem cells in the culture after multiple harvesting cycles may be mixed and tested for functionality by quality control of relevant biomarkers. These cells may be frozen prior to therapeutic use.

Table 1 compares the number of cells obtained from the methods of the present invention and from methods of the prior art.

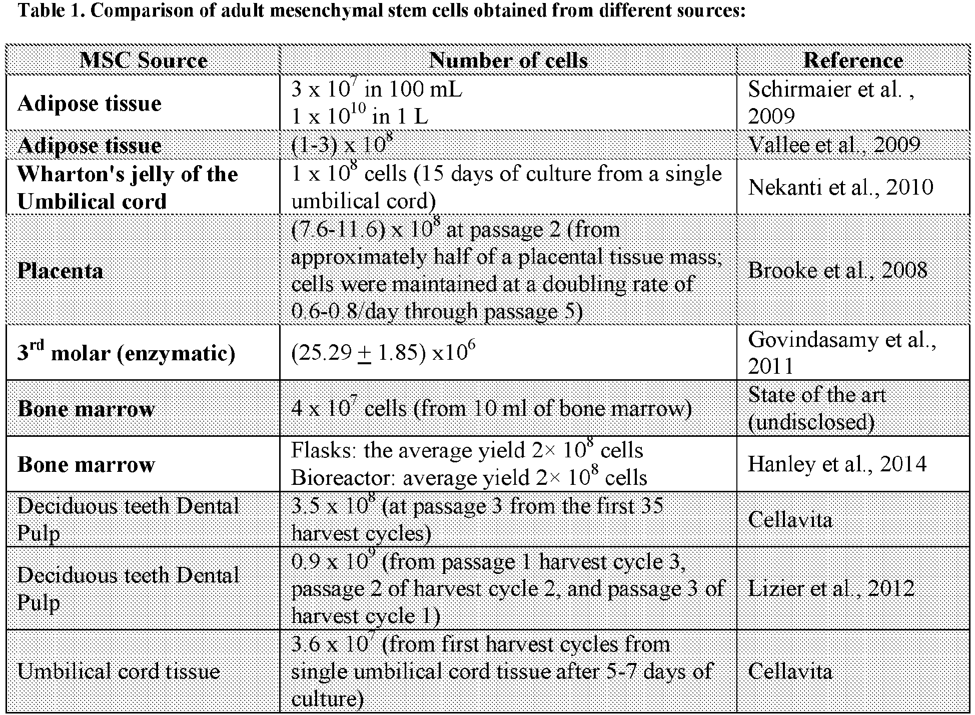

In one embodiment, the method of the invention comprises washing excess blood from the tissue and disinfecting the collected tissue with antibiotic and buffer solutions. The tissue is also mechanically dissociated into tissue fragments, wherein the tissue is cut into pieces visible to the eye without microscope. For example, the tissue fragments should be greater than 1 mm. The tissue fragments are exposed to supplemented culture medium. In preferred embodiments, the supplemented culture medium is based on DMEM, more preferably DMEM F12. After a few of culturing, when the adhered cells reach semi-confluence, the tissue fragment is transferred to a new plate for another harvest cycle.

In some embodiments, the stem cells after each harvest cycle (P0) are cultured in multiple passages to expand the number of stem cells in the culture. The stem cells may be passaged for three, fifteen, or 50 passages while maintaining a normal karyotype. In some embodiments, in each repeat cycle the stem cells are obtained from the tissue pieces mixed from several donors or from several samplings from the same donors (e.g. deciduous pulps from same donor, different tissue samplings through surgical procedures) of the preceding repeat cycle. The sourcing tissue can be obtained fresh or thawed after cell banking, cryopreservation.

Subsequent to explant harvesting, enzymatic splitting/passaging of the cells undifferentiated or differentiated can be performed in classic cell culture conditions, known in the state of the art, or under bioreactor conditions, in sterile plastic flasks, glass ware, sterile bags, micro-carries, microfibers reactors, etc.

In one embodiment, the method of expansion of stem cells may be accelerated by mechanical transferring, nutrient deprivation, and hypoxic conditions. In some implementations, the method comprises the following steps:
(i) obtaining postnatal, young or adult mesenchymal cells and precursors stem cells from the **minimally manipulated tissue** (preferably isolated from tissue containing cells of neuro-ecto-dermal originated stem cell niches and pericytes, or/and epithelial source i.e. dental tissue, post-delivery tissue , such as placenta, amniotic, umbilical cord; hair follicle, and adipose tissue of under-ectodermal source; etc.
(ii) wherein the said tissue is essentially tested for viability, maintained with antibiotic, antimicotic conditions, cleaned substantially from blood, fractionated mechanically to small pieces (preferably about 0.5-5 mm in order to expose to supplement medium).
(iii) Said stem cells colony formation from minimally manipulated tissue is performed under nonenzymatic/ mechanical method.
(iv) Said colony formation is essentially induced by mechanical manipulation and medium exposure and cell adhesion to plastic ware, bead or adhesion surface, wherein said colony initiation starts **about day** 3 or later under hypoxic/ starvation stress conditions low serum medium 5% or less, **or later at normal culture growth conditions.**
(v) Repeated multiple harvesting offer use of low passage number with high cell number, wherein each harvest initiate new primary culture, wherein after initial harvest tissue diminish in size, stressed and duration cell harvesting from subsequent harvests is faster **about two folds** than at initial primary harvesting culture.
(vi) Said cells obtained from such minimally manipulated tissue express same markers in vivo and in vitro originated from multiple stem cell niches which mimic in vivo conditions.
(vii) The term for said colony formation may be induced and accelerated substantially at least two folds under stress- hypoxic conditions that can be induced by chemical stress, for example hypoxic, mechanical stress, for example under coverslip pressure; or/and supplement starvation, preferably serum 5% and less (see Example MS2 below).
(viii) After colony forming units from primary harvest of stem cells from multiple repeated harvested are passaged enzymatically or non-enzymatically exposed to conditions suitable for cell proliferation, wherein in preferable example serum supplementation is about 15%, or growth factors and additional supplementation performed. Said proliferation may be accelerated significantly if used low glucose DMEM which is well known in the state of the art to accelerate hypoxic/ stress conditions (see example MS3 below).

Transplantation for cell therapy may be performed within buffered pharmaceutical composition, hydrogel, implantation scaffold, with or without additional active additive or synergistic pharmaceutical or biological ingredients, etc.

Obtained cells can be used for various cell therapy applications by administrating cells though systemic, or/and local administration route though single but preferably repeated mode of administration as shown in the example below in dogs treatment of multiple sclerosis like treatment -when the optimal results obtained through two-three, preferably >two, three or four repeated injections of cells to improve the symptoms. In more preferable approach, cells should be administered at relatively early stage of the disease when reversibility of the process can be modulated by cell therapy.

The above listed advantages will be translated into multiple customers' benefits:
- It is expected to allow for robust and reproducible industrial-scale production of well defined and characterized stem cells
- Higher quantity of starting materials from early passage for future bioreactor expansion
- Unique protein expression pattern of Cellavita cells allows for easy and standardized characterization

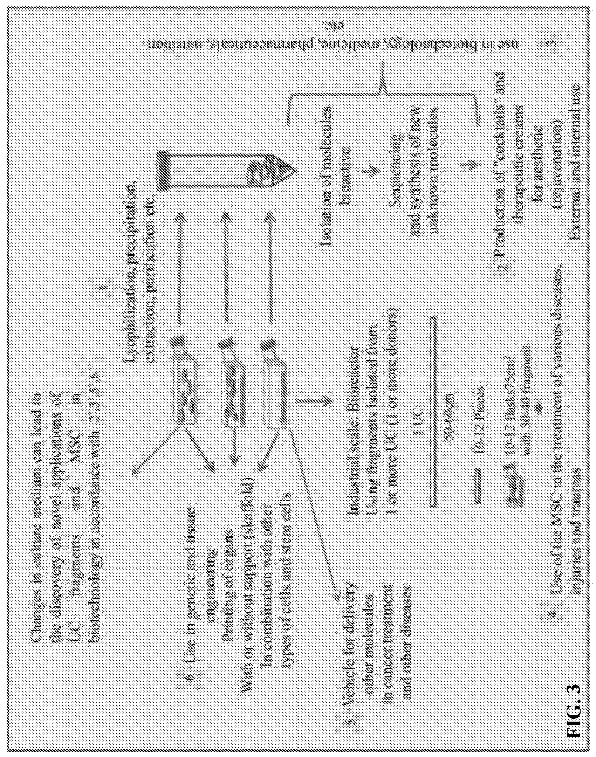

Possible applications of non-enzymatic method of stem cells isolation and mechanical transfer of fragments are presented on FIG. 3. The cells or isolated fragments may be lyophilized, precipitated, extracted, and purified or further processed for isolation of bioactive molecules. These molecules can be used as a template for sequencing and synthesis of new unknown molecules in order to create "cocktails" or therapeutic creams for aesthetic (rejuvenation) external and internal use. On the other hand the MSC can be used in the treatment of various diseases, injuries and traumas as stem cell therapy. These cells further can serve as vehicles for delivery diverse therapeutic or suicide molecules in cancer treatment. These cells can be also used in genetic and tissue engineering, printing of organs with or without support (or scaffold), and in combination with other types of cells and/or stem cells in multiple diseases.

However, we discovered the final population of isolated stem cells can present at the same time similar but also definite properties as demonstrated on Table 2 according with stem cell niches, which can influence stem cells properties.

**Table 2. Different stem cell niches in UC and DP tissues**

| STEM CELL NICHES IN UMBILICAL CORD (UC) TISSUE | | | STEM CELL NICHES IN DENTAL PULP (DP) TISSUE |
|---|---|---|---|
| Derived from extra-embryonic mesoderm | | | Derived from embryonic ectoderm |
| Absent in UC | | | Nerve networks in nerve plexus |
| Wharton jelly | | | |
| Gelatinous substance made up of mucopolysaccharides (hyaluronic acid and chondroitin sulfate) | | | Absent in DP |
| Contains some fibroblasts and macrophages | | | |
| Absent in UC | | | Dental pulp soft connective tissue, capillaries, lymphatic and nervous elements |
| | | | Contains fibroblasts and undifferentiated mesenchymal cells |
| | | | Contains collagen fibers, type I and II |
| | | | |
| Absent in UC | | | Small capillaries |
| Vein and Arteries | | | Absent in DP |
| inner longitudinal smooth muscle | outer circular | vascular endothelium cells | Absent in DP |
| | smooth muscle | | |
| Absent in UC | | | Subodontoblast region |
| | | | Contains stem cells - odontoblasts |
| Amniotic epithelium | | | Absent in DP |
| Basal layer | | | |
| Sub-amniotic epithelium | | | Absent in DP |

Though Table 2 presents examples for two tissues, the disclosure is not limited to these tissues and can include any type of the tissues:
a. derived from embryonic ectoderm and neural crest (sublingual and submandibular tissue, salivary and lacrimal glands, dental papilla, dental lamina, Hertwig's epithelial root sheath, dental follicle, periodontal ligament, skin, birthmark and others);
b. derived from extraembryonic mesoderm (yolk sac and amnion, umbilical cord, placenta and fetal membranes); and
c. derived from embryonic mesoderm (adipose tissue and bone marrow).

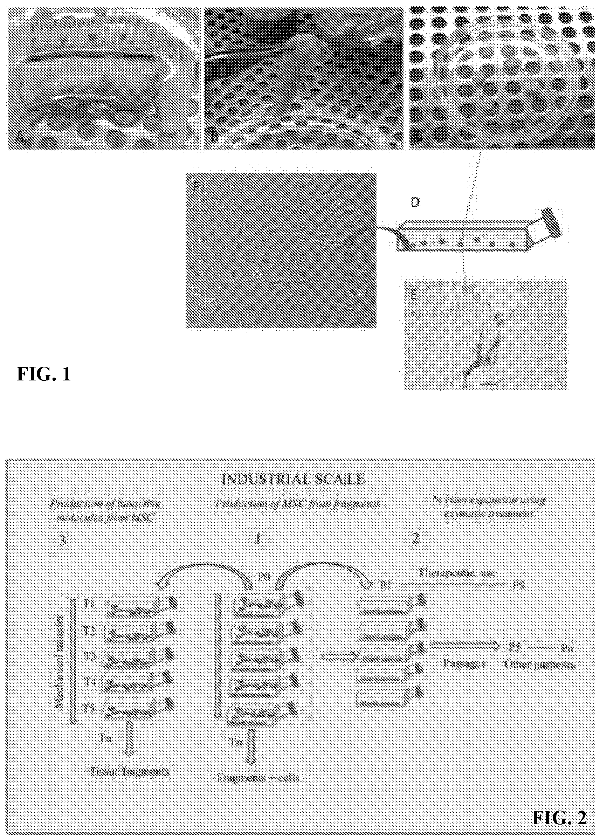

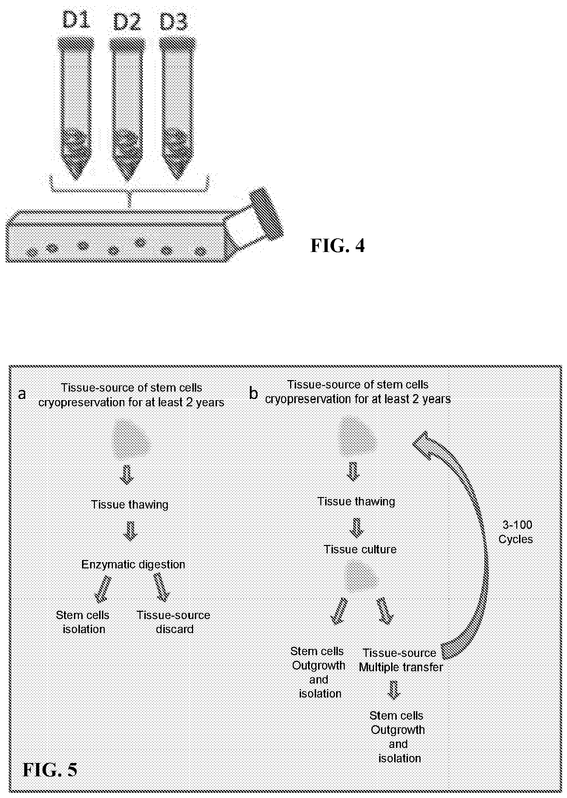

All these tissues can serve as a source of the fragments, which can be processed according to Figure 1 and 2, and used according to Figure 3 and 4.

Each stem cell types obtained from different tissue sources, described in a, b, c but not limited by these examples, can be used in Cell Therapy and Regenerative Medicine for various purposes and for multiple indications. At the same time once these stem cells are of different embryonic origin, they can be used in combination in order to provide the enhanced or the synergic or the complementary effect in therapeutic treatment. Exemplary tissue sources include: umbilical cord, dental/peri-dental tissue, mandibular tissue, mucosal tissue, gastro-intestinal tissue, placenta, amniotic fluids or amniotic sac, neuronal tissue (for example nerve or glial cells-containing tissue), tissue derived from neuronal crest or peripheral nerve system origin, muscle, bone, skin (dermal, epidermal, keratinocytes-containing, or melanocyte-containing tissue, foreskin, plastic surgery or biopsy-derived skin), adipose/fat tissue, blood and other body fluids, mammary tissue, endothelial tissue, testicular tissue, ovary, cardiac tissue, liver, bone tissue. lung tissue, salivary gland tissue, pancreatic tissue, heart, and spleen.

Non-enzymatic method of stem cells isolation and mechanical transfer can be also extended for other tissues with minimal variation, such as umbilical cord (FIG. 1). For example, a 5 cm piece from umbilical cord (UC) is isolated (FIG. 1A) washed to in order to eliminate the excess blood excess (FIG. 1B) with sterile solution like saline solution. The initial piece of UC tissue is cut into even smaller tissue fragments for culturing (panel C) and is mechanically transferred into basal medium (FIG. ID). The basal medium may be specific for each type of stem cells, and its specific formulation depends on the tissue origin. Stem cells positive for CD105 migrate from the tissue in order to adhere onto the culture surface (FIG. IE). In FIG. IF, the culture of mesenchymal stem cells (MSC) originated from the small UC tissue fragments can be observed.

This method can be easily converted into industrial scale culturing as shown in FIG. 2. Multiple fragments are isolated from UC and placed into various culture flasks for MSC production. Following in vitro expansion using enzymatic treatment these cells can be used in Cell Therapy and for other purposes. Several mechanical transfers of UC fragments can provide great quantities of MSC, which can be used for production of bioactive molecules.

Another embodiment of our invention is directed to a treatment regimen for patient involving stem cells isolated according to this method. The regime begins with systemic administration of the stem cells to protectively modulate immune and inflammation status of the patient. When anti-inflammatory immunomodualtory effect is confirmed by measuring of cytokines and immunoglobins and other immunomodulatory factors in patient (e.g. with blood tests), then local implantation of the stem cells can be applied for regenerative cell therapy.

Functional assay may include characterization of synergistic factors, such as for example anti-inflammatory, antimicrobial and regenerative for disease that can have a risk of infection, such as hospital infection, wound healing, such as diabetic ulcers, surgery wounds, etc.

In preferable embodiment Cellavita technology using cell therapy treatment should be done adjunctively with other accepted medical and supportive treatments patient undergo, but in vitro compatibility of adjunctive medication should be tested no to inhibit cell immuno-modulating and regenerative potential. For example, antibiotics, steroids, anti-proliferative and immunosuppressive agents can be toxic, or inhibiting cells activity, therefore the regimen should be adjusted to relevant therapy regimen.

This disclosure, its aspects and implementations, are not limited to the specific material types, components, methods, or other examples disclosed herein. Many additional material types, components, methods, and procedures known in the art are contemplated for use with particular implementations from this disclosure. Accordingly, for example, although particular implementations are disclosed, such implementations and implementing components may comprise any components, models, types, materials, cells, versions, quantities, and/or the like as is known in the art for such systems and implementing components, consistent with the intended operation.

The words "exemplary," "example," or various forms thereof are used herein to mean serving as an example, instance, or illustration. Any aspect or design described herein as "exemplary" or as an "example" is not necessarily to be construed as preferred or advantageous over other aspects or designs. Furthermore, examples are provided solely for purposes of clarity and understanding and are not meant to limit or restrict the disclosed subject matter or relevant portions of this disclosure in any manner. It is to be appreciated that a myriad of additional or alternate examples of varying scope could have been presented, but have been omitted for purposes of brevity.

While this disclosure includes embodiments of many different forms, there is shown in the drawings and will herein be described in detail particular embodiments with the understanding that the present disclosure is to be considered as an exemplification of the principles of the disclosed methods and systems, and is not intended to limit the broad aspect of the disclosed concepts to the embodiments illustrated.

### EXAMPLES

### Example 1: Viability of Dental Pulps

Maher Al-Atari Abou-Asi et al. (2011) claimed that the origin of pluripotent cells extracted from dental pulp (DP) according to the publication of Kerkis et al., 2006, cannot be dental pulp, because the patients aged between 5 and 7 years do not have dental pulp, but rather what is referred to as dental germ, which is an aggregation of undifferentiated cells which will form the future tooth with its different parts: enamel, pulp, gum, cementum, bone, blood vessel and nerve. However, Cordeiro et al. (2008) found that stem cells from human exfoliated deciduous teeth generate a DP-like tissue *in vivo.* The authors transplanted SHED seeded in biodegradable scaffolds prepared within human tooth slices into immunodeficient mice. They observed that the tissue formed by these cells, demonstrated architecture and cellularity that closely resemble those of a physiologic DP. Ultrastructural analysis with transmission electron microscopy and immunohistochemistry for dentin sialoprotein suggested that SHED differentiated into odontoblast-like cells *in vivo.* Notably, SHED also differentiated into endothelial-like cells, as demonstrated by β-galactosidase staining of cells lining the walls of blood-containing vessels in tissues engineered with SHED that is stably transduced with LacZ. This work suggests that exfoliated deciduous teeth constitute a viable source of stem cells for DP tissue engineering.

We screened viable pulps prior to cells cultivation based on the color of the pulp at the time of extraction. The tooth exfoliated should have pulp red in color as in indication of cell viability. The red color indicates that the pulp received blood flow up until the time of removal. If the pulp is gray in color, it is likely that blood flow to the pulp has been compromised. Thus, the stem cells are likely necrotic and are no longer viable for recovery (Arora et al., 2009).

Example of pulp extraction and cultivation performed form freshly extracted deciduous tooth from a healthy subject after receiving Informed Concern approved by ethical committee (Helsinki in Europe, IBR in USA) washed repeatedly in sterile solution containing 50% pen/strep solution (100 units/ml penicillin, 100 units/ml streptomycin) and 50% Phosphate Buffered Saline (PBS), freshly extracted pulp washed again and viability testing of pulp performed in DP Tissue Maintenance Medium supplemented with 3-20% fetal bovine serum (FBS), 100 units/mL penicillin, 100 units/ mL streptomycin. Once the colony formation is initiated from minimally manipulated dental pulp and cells are attached to the plastic cell culture surface, safety tests are performed (sterility test and mycoplasma test) before subsequent harvest cycles repeated and or cells expansion through passaging and or cryopreservation of tissue or cells.

Pulp extraction and cultivation performed as follows:
- A freshly extracted deciduous tooth from a healthy subject washed repeatedly in sterile solution containing 50% pen/strep solution (100 units/ml penicillin, 100 units/ml streptomycin ) and 50% Phosphate Buffered Saline (PBS).
- DP removed from the tooth with the aid of a sterile needle. Freshly extracted pulp washed in a solution containing 3% pen/strep solution. Viability testing of pulp performed in DP Tissue Maintenance Medium supplemented with 15% fetal bovine serum (FBS, Hyclone), 100 units/mL penicillin, 100 units/ mL streptomycin, 2 mM L-glutamine, and 2 mM nonessential amino acids.
- Once the dental pulp becomes attached to the plastic cell culture surface, colony formation will be observed from 5 days to two weeks.
- Safety tests performed are sterility test and mycoplasma test.

### Example 2: Stem cells from dental pulp explants

Different examples of dental tissue detailed in the recent review of dental origin derived stem cells (Deepa Ponnaiyan 2014) describe different characteristic of biomarkers of stem cells from dental tissues, such as dental pulp stem cells, periodontal ligament stem cells, stem cells from human exfoliated deciduous teeth, dental follicle progenitor cells stem cells from apical papilla, oral periosteum stem cells and recently from gingival connective tissue. Remarkably, most of the markers are similar between different dental stem cells, but Notch and CD29 markers different in expression between stem cells from various dental sources. The marker expression *in vitro* and *in vivo* of cells obtained by our disclosed method is the same.

### Example 3: Teratoma Formation Human IDPSC From Repeated Harvesting at Early Passage

Teratomas formation is an essential tool in determining the pluripotency of cells, such as embryonic stem cells (ES cells) or induced pluripotent stem cells (iPS cells). A consistent protocol for assessment of teratoma forming ability of the cells similar to protocol published recently by Gropp et al. (2012) was used in our studies. Our method was shown to be highly reproducible and efficient when 10⁶ cells (Gropp et al. used 10⁵ cells) of mouse ES cells and human iPS cells were subcutaneously co-transplanted with Matrigel into immunodeficient mice. In 100% of cases, we observed teratoma formation in a large number of animals and even in follow-up examination up to 6 months after transplantation. We used this method for bio-safety analysis of other adult mesenchymal stem cells (MSC), such as those derived from dental pulp of deciduous teeth, umbilical cord, adipose tissue, and others. In addition to the experimental systems described here, this method was validated using dog fetal MSC from bone marrow, liver, yolk sac, allantois and amniotic liquid which also partly express pluripotent markers.

### Methods for the Teratoma Assay:

The development of tumors was monitored for 4 month (~16 weeks). For pluripotent cells, the histological criteria for teratomas were the development of tissues derived from all three germ layers. This analysis was performed by a pathologist. For adult/mesenchymal stem cells, any type or any changes on normal tissue integrity in the site of cell injection were taken in consideration.

### Results

### THE EXPERIMENTAL SYSTEMS:

a. Mouse embryonic stem cells
b. Mouse 3T3 fibroblasts
c. Permanent mouse cell line Balbc 3T3 cell line, clone A31
d. Human iPS-IDPSC
e. Human ES cells
f. Human IDPSCs

We used aforementioned method in diverse studies to characterize different mouse ES cell lines pluripotency established by us as well as to confirm ES cells pluripotency at passage 25 higher and for characterization of sub-clones obtained from mouse ES cell lines (Sukoyan et al., 2002; Carta et al., 2006; Kerkis et al., 2007; Lavagnolli et al., 2007; Hayshi et al., 2010). Additionally, this method was used to characterize the pluripotency of iPS cells derived from immature dental pulp stem cells (IDPSC) in more recent publication of our group (Beltrão-Braga et al., 2011). In this publication, the human IDPSC were used as a control for iPS-IDPSCs. We showed that iPS-IDPSCs formed teratomas with tissues originated from all three germ layers, while IDPSC were not able to produce any type of teratomas or any other type of neoplasms.

### THE EXPERIMENTAL SYSTEMS:

a. IDPSC three different primary cultures at early (n=10) and late passages (n=10)
b. Human primary fibroblast

We previously reported the isolation of a population of IDPSCs that express embryonic stem cell markers Oct-4, Nanog, SSEA-3, SSEA-4, TRA-1-60, and TRA-1-81 as well as several mesenchymal stem cell markers during at least the first 25 passages while maintaining a normal karyotype and the characteristic rate of expansion of stem cells. Moreover, these cells can be induced *in vitro* to undergo uniform differentiation into smooth and skeletal muscles, neurons, cartilage, and bone under chemically defined culture conditions. Although IDPSCs have a small size and a paucity of cell organelles in the cytoplasm, they are different from naive pluripotent cells presenting typical mesenchymal/fibroblast-like morphology (Lizier et al., 2012). IDPSC are mesenchymal in contrast to ES and iPS cells, which epithelial (Figure 5). The principle difference between MSC cells and ES cells (or iPS cells) is that MSC are migrating, plastic, and anchoring. MSC cells synthetize extracellular matrix and are cell junction free cells.

The tumor formation is related with aforementioned ES and iPS cells, but not with normal MSC. The IDPSC are composed by population of MSC with a variable number of stem cells expressing pluripotent markers (1-25% of cells) (Kerkis et al., 2006; Lizier et al., 2012). This protocol was adapted for population of IDPSC, especially in respect of cell number used, which was calculated on the basis that 20% of IDPSC express pluripotent markers. Rather than testing 10⁶ cells, teratogenicity of IDPSC was assessed with 5×10⁶. Although the presence of IDPSC DNA was found within all studied organs, no tumor formation or any morphological changes were observed (Lizier et al.)

### Example 4: Expression pattern of principal stem cell markers is not influenced by cryopreservation of Dental Pulp (DP)

Due to the immature nature of the newborn cells from umbilical cord, amniotic sac, placenta and young stem cells from dental pulp, they show a higher proliferative capacity and higher expansion rate when compared with their adult counterparts (bone marrow and adipose tissue). So cells from umbilical cord, amniotic sac, placenta, and dental pulp can be cryopreserved for future use as an allogenic therapy (the cryopreservation of tissue fragments and/or cells done initially at freezer (about -80 °C) and subsequently in liquid nitrogen (about -196 °C).

We found that cells harvested before and after cryopreservation and thawing of explants maintain same pattern of factors expression. We have shown that cells derived from minimally manipulated explants under repeated harvesting long term culture from dental pulp and umbilical cord pre-and post- cryopreservation, express mesenchymal markers (i.e., CD 73, CD 105, CD90), do not express hematopoietic markers (i.e. CD34, CD45), and do not express at least one histocompatibility marker- HLA-DR, and maintain karyotype stability, maintain multipotency (differentiate to different lineages including neuronal under culturing with inducing factors, such as retinoic acid, BMP- TNF, NGF, etc.)

For example, dental pulp, removed from deciduous tooth was cryopreserved and thereafter thawed for isolation of IDPSCs. IDPSCs were cultivated until Harvest 0/Passage 3 (H0P3) and compared by FACS analysis to the hIDPSC from the same passage received from fresh DP. The expression pattern of both cells found to be similar (Table 3). Both kinds of IDPSCs were positive for the expression of mesenchymal markers (CD105, CD73, CD90) and negative for the expression of hematopoietic marker CD45 and histocompatibility marker HLA-DR.

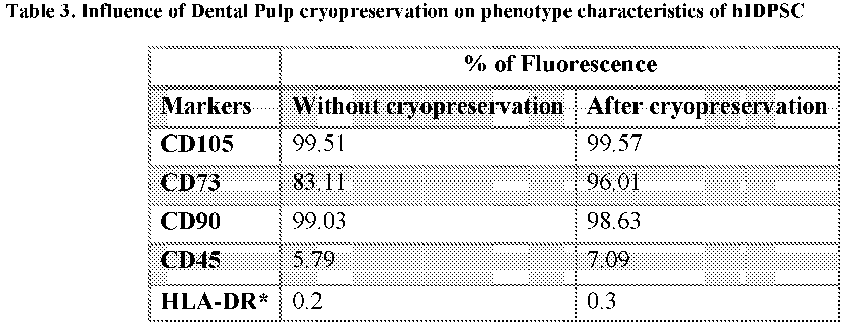

### Example 5: Influence of growth medium on growth rate of hIDPSC

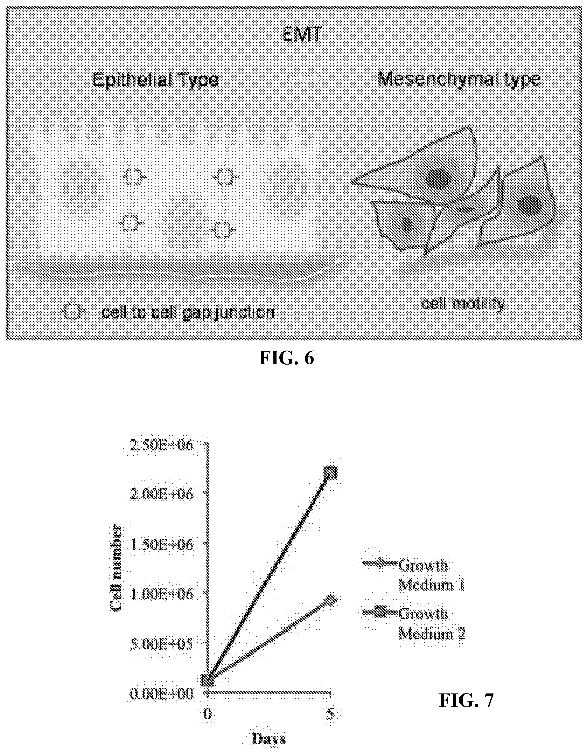

To evaluate optimal growth conditions for cultivating hIDPSC, equal numbers of cells were seeded and cultured for 5 days in two types of growth medium (Table 4). Cell growth rate found to be higher (twice) in type 2 growth medium (FIG. 7), so growth medium composed of DMEM-low glucose and supplemented with 15% of FBS is more optimal for cells cultivation.

**Table 4. Composition of various medium used**

| Tissue Maintaining Medium | Medium 1 (Growth & Tissue Maintaining) | Medium 2 (Growth) |
|---|---|---|
| DMEM-F12 | DMEM-F12 | DMEM-Low glucose |
| FBS <5% | FBS >10% (e.g. 15%) | FBS >10% (e.g. 15%) |
| 100 units/ml penicillin, | 100 units/ml penicillin, | 100 units/ml penicillin, |
| 100 units/ml streptomycin | 100 units/ml streptomycin | 100 units/ml streptomycin |
| 2 mM L-glutamine | 2 mM L-glutamine | 2 mM L-glutamine |
| | 2 mM nonessential amino acids | -- |

### Example 5: Initiation of cell sprouting from Dental Pulp (DP)

Two equal fragments of DP were maintained in tissue culture. One of the fragments was cultured in the presence of 15% FBS, and the other was cultivated under serum starvation stress conditions (4.8% FBS). Initiation of cell sprouting from DP was 3 days in the presence of 4.8% FBS and 7 days in the presence of 15% FBS. Thus, serum starvation can reduce the time required for pulp transfer. Thus initiation of cells sprouting from dental pulp was faster in presence of serum starvation medium.

### Example 7: Paracrine effects of Late Population (LP) hIDPSCs include anti-inflammation, immunomodulation, and antimicrobial properties

MSCs assist via paracrine mechanisms and modulate the regenerative environment via anti-inflammatory and immunomodulatory mechanisms.

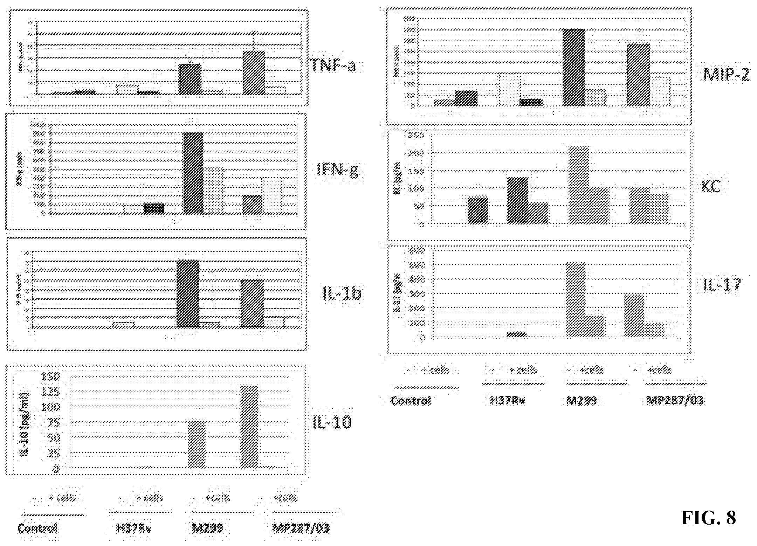

Effect of IDPSCs intraperitoneal inoculation on cytokine production by lung cells of C57Bl/6 mice intravenously infected with *Mycobacteria tuberculosis (Mtb)* and *Mycobacteriaum bovis* strains differed in virulence. As shown in the BIOPLEX test data, IDPSCs inoculation reduced production of cytokines by the infected lung cells (FIG. 8). Strong reduction of proinflammatory (TNF-a, IL-1b, MIP-2, IL-17) and anti-inflammatory (IL-10) cytokines was observed in the lungs infected with highly virulent Mtb strains. Reduction of IFN-g and KC production was less pronounced. Induction of IL-4 production was observed only in the mice inoculated with IDPSCs.

**Tumor necrosis factor** (TNF, cachexin, or cachectin, and formerly known as tumor necrosis factor alpha or TNFα) is an adipokine involved in systemic inflammation. The primary role of TNF is in the regulation of immune cells. TNF, being an endogenous pyrogen, is able to induce fever, apoptotic cell death, cachexia, inflammation and to inhibit tumorigenesis and viral replication and respond to sepsis via IL1 & IL6 producing cells. Dysregulation of TNF production has been implicated in a variety of human diseases including Alzheimer's disease.

**Interferon gamma** (IFNγ or IFN-g) is a dimerized soluble cytokine that is the only member of the type II class of interferon, is known as immune interferon a cytokine that is critical for innate and adaptive immunity against viral and intracellular bacterial infections and for tumor control. IFNγ is an important activator of macrophages. Aberrant IFNγ expression is associated with a number of inflammatory and autoimmune diseases. The importance of IFNγ in the immune system stems in part from the ability of IFNγ to inhibit viral replication directly and most importantly from IFNy's immunostimulatory and immunomodulatory effects.

**Interleukin 17** is a cytokine that acts as a potent mediator in delayed-type reactions by increasing chemokine production in various tissues to recruit monocytes and neutrophils to the site of inflammation, similar to IFNγ. Interleukin 17 is a proinflammatory cytokine that responds to the invasion of the immune system by extracellular pathogens and induces destruction of the pathogen's cellular matrix.

**Interleukin-1 beta (IL-1β)** also known as catabolin, is a cytokine protein that is an important mediator of the inflammatory response. It is involved in a variety of cellular activities, including cell proliferation, differentiation, and apoptosis. The most notable role of IL-17 is its involvement in inducing and mediating proinflammatory responses. IL-17 is commonly associated with allergic responses.

**Interleukin-10 (IL-10),** also known as human cytokine synthesis inhibitory factor (CSIF), is an anti-inflammatory cytokine. IL-10 is a cytokine with pleiotropic effects in immunoregulation and inflammation.

Chemokine ligand 2 (CXCL2), also called macrophage inflammatory protein 2 **(MIP-2),** plays a major role in mediating the neutrophilic inflammatory response and a mediator in the development of sepsis. **KC** and macrophage-inflammatory protein-2 **(MIP-2)** are chemokines with the C-X-C motif that exhibit distinct temporal patterns of expression in the skin following surgical injury. KC and MIP-2 are neutrophil chemoattractants. Both chemokines are known to be expressed in a broad spectrum of acute and chronic inflammatory settings and are believed to be critical determinants of the nature and magnitude of the ensuing inflammatory reaction.

### Example 8: Robust immunomodulatory capacity of hIDPSC obtained by multiple harvesting (LP) of minimally manipulated tissue

IDPSC demonstrate immuno-modulatory action when co-cultured derived from monocytes-derived dendritic cells. Co-culture resulted in reduced capacity of Lin T proliferation (50% and more) and induced Lin T differentiation towards significant increase of proportion of Lin T CD4⁺/FoxP3⁺/IL10⁺ and Lin T CD4⁺/FoxP3⁺/IFN-γ⁺ cells. No effect of HLA-DR expression in IDPSCs was observed.

We have proven that although stem cells obtained from minimally manipulated tissue repeatedly harvested (Cellavita method) express most pluripotent markers, the injection of these stem cells into mice does not form teratomas.

**Table 5. Comparison of hIDPSC treatment between various mammalian species, models using fresh culture derived cells and cells form cryopreserved pulps and cells.**

| Subject | Type of injury or disease | Weight | Administration | Number of transplants | Number of transplanted | Efficiency scope 0-5 |
|---|---|---|---|---|---|---|
| **New cell therapy treatment examples with hIDPSCs cells from cryopreserved pulps and cells at low passages (passages 3-4)** | | | | | | |
| Dog (n = 30) | Dempster (human multiple sclerosis) | a. 3-5 kg | Endogenous (EV) | a. 1-2 | a. 4 x 10⁶ | 2-5* |
| | | | | b. 2-3 | b. 6 x 10⁶ | * all subjects alive with no reverse of function: |
| | | b. 5-20 kg | | c. 4 | c. 4 x 10⁶ | |
| | | | | | | - 40% cured (3-4) |
| | | c. > 20 kg | | | | - 50% significant functional recovery (4-5) |
| | | | | | | - 10% poor functional recovery, which improved with time (2-3) |
| Human (n = 5) | Bone regeneration, dental implant | avg 70 kg | Local (with scaffold) | 1 | 2 x 10⁶ | 5 |
| | | | | | | Bone regeneration, maturation and Neovascularization |

| **Treatment with Fresh cells (published previouslv 2006-2011)** | | | | | | |
|---|---|---|---|---|---|---|
| Mice (n = 16) | Spinal cord injury | 25-30 g | Epicenter of the lesion | 1 | 8 x 10⁵ | 4 |
| | | | | | | White matter regeneration and partial functional recovery |
| Rat (n = 8) | 5 mm x 8 mm cranial bone defect | 320-420 g | Local (with scaffold) | 1 | 1 x 10⁶ | 5 |
| | | | | | | Bone reconstruction |
| Rabbit (n = 15) | Corneal defect 6.0-10.0 mm in diameter | - | Local (with scaffold) | 1 | 1 x 10⁶ | 3-4 |
| | | | | | | Corneal reconstruction Functional recovery in 50% of animals (Scope depends on grave of injury) |
| Ovine (n = 4) | Osteonecrosis of femoral head | 35 kg | Local | 1 | 1x10⁶ | 5 |
| | | | | | | Bone regeneration, functional recovery |

### Example 9: Discovery of novel nerve plexus and subodontoblastic stem cell niches in dental pulp

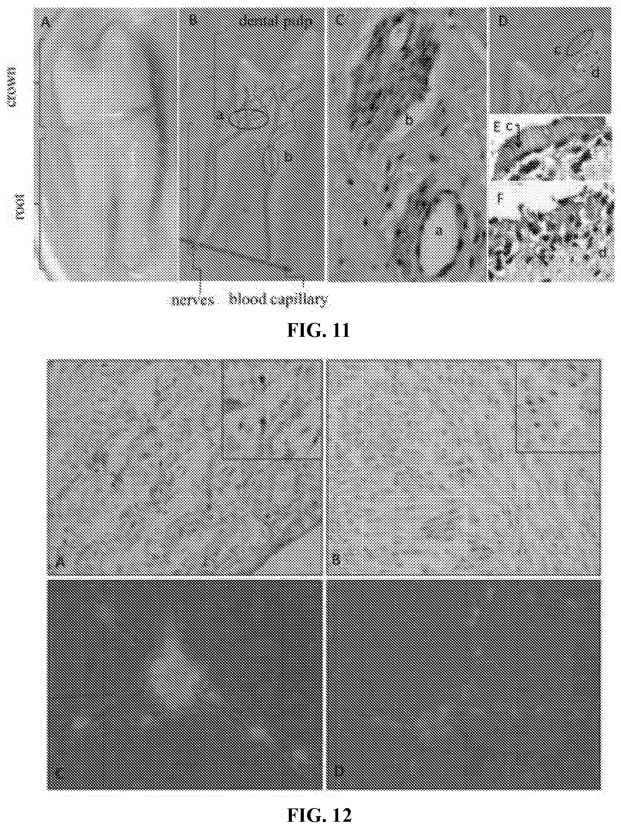

Immunostaining for early neuronal marker nestin showed a difference in expression between the nerve plexus, perivascular niche, and subodontoblastic niche. Cells in the nerve plexus were positive for nestin (FIG. 11C, b) while cells in the perivascular niche were negative for nestin (FIG. 11C, a). Cells in the subodontoblastic niche were also positive for nestin (FIG. 11D, c and 11E, c), though the cell-free and cell-rich zone niches were negative for nestin (FIG 11D, d, and 11F, d)

### Example 10: Discovery of putative niches of stem cells in umbilical cord

Putative stem cell niches in umbilical cord were detected by detecting neurofilaments (NF) and low-affinity nerve growth factor receptor (p75 or CD271). NF is 10 nm filaments or intermediate filaments found in neurons. CD271 is one of two receptor types for neurotrophins, which are a family of protein growth factors that stimulate neuronal cells to survive and differentiate. Both NF and CD271 were found in umbilical cord tissue. The expression of NF was primarily in the subamniotic epithelium zone (FIG. 12A) while the expression of CD271 was primarily in the vein and arteries (FIG. 12B). The stem cells isolated from these niches were able to undergo neuronal differentiation *in vitro* expressing beta-III tubulin and glial fibrillary acidic protein (GFAP) (FIG. 12C and 12D).

### Example 11: Discovery of pluripotent stem cell or immature precursor niches in umbilical cord

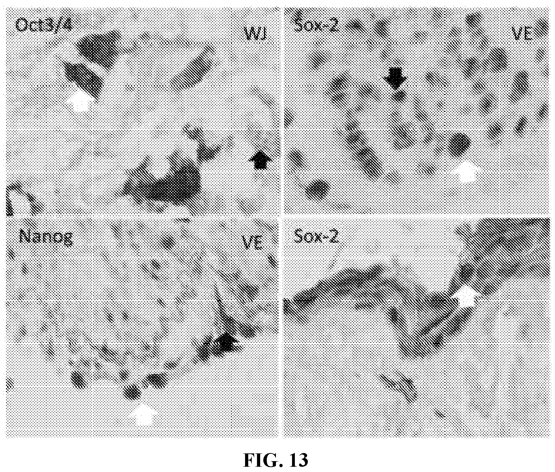

The expression of transcription factors, such as Oct 3/4, nanog, and Sox2, were detected. Oct 3/4 protein expression was appropriately detected with a nuclear localization in the cells of WJ, veins (VE), arteries (AR), and in cells localized the basal layer (BL) of amniotic epithelium (AEP) (FIG. 13). A small amount of the cell expressing all three of these markers in the nucleus, similar to pluripotent stem cells, was detected. Therefore, we can predict the isolation o a small number of "genuine" pluripotent stem cells from the VE or AR and from the AEP of umbilical cord. These cells may also be found in other related tissues, such as the placenta and tissue derived from the extraembryonic mesoderm.

### Example 12: Safety assay ― stem cell niches in vitro cultivation following several mechanical transfers

It is common knowledge that stem cells in located in particular anatomical sites of the human body, which are called stem cell niches (SCN). Each tissue has its own SCN, which, in response to signals received from the surrounding microenvironments, makes a decision about the fate of the stem cells. However, little is known about *in situ* biological characteristics of stem cells within their natural niches, and how these characteristics can change during *in vitro* culture of MSC. For example, the cells can stop expressing some proteins or start expressing new proteins. This question is very important, because it can have far-reaching implications in clinical studies and can explain the successes and/or failures of stem cells in regenerative medicine. The cells cultured *in vitro* lack tissue architecture as well as all of the functions based on tissue context (cell-to-cell interaction, secretion of specific factors, etc.). That is why cell lines can lose tissue-specific functions and may acquire a molecular phenotype quite different from the cells in SCN. The investigation of expression pattern of stem cell markers within the SCN is essential for the evaluation of stem cell safety, especially for their use in humans.

In Table 6, we summarized the data obtained by immunohistochemistry assay for *in vivo* tissue samples of cells after isolation and flow cytometry for *in vitro* samples of cells cultivated using several mechanical transfers. Table 6 demonstrate that the majority of markers expressed by stem cells *in vivo* and *in vitro,* especially dental pulp, did not show lack of markers in isolated cells obtained following mechanical transfer. The cells obtained following mechanical transfer of umbilical cord tissue demonstrated slight differences in the frequency of cells expressing c-kit and vimentin. Though these results are from stem cells isolated from dental pulp and umbilical cord tissue, such safety assay can be used with stem cells from other sources, even in for cells in liquid phase.

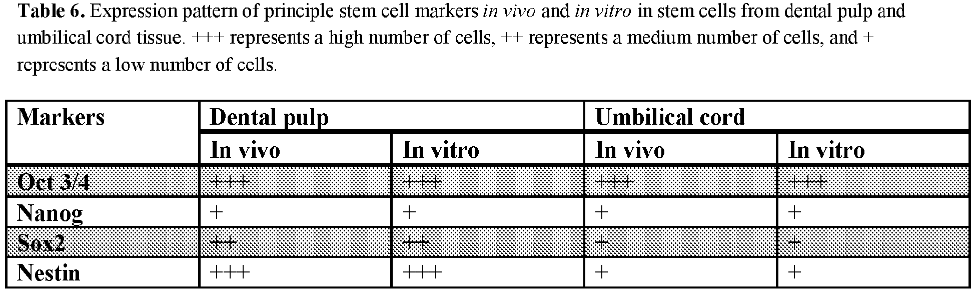

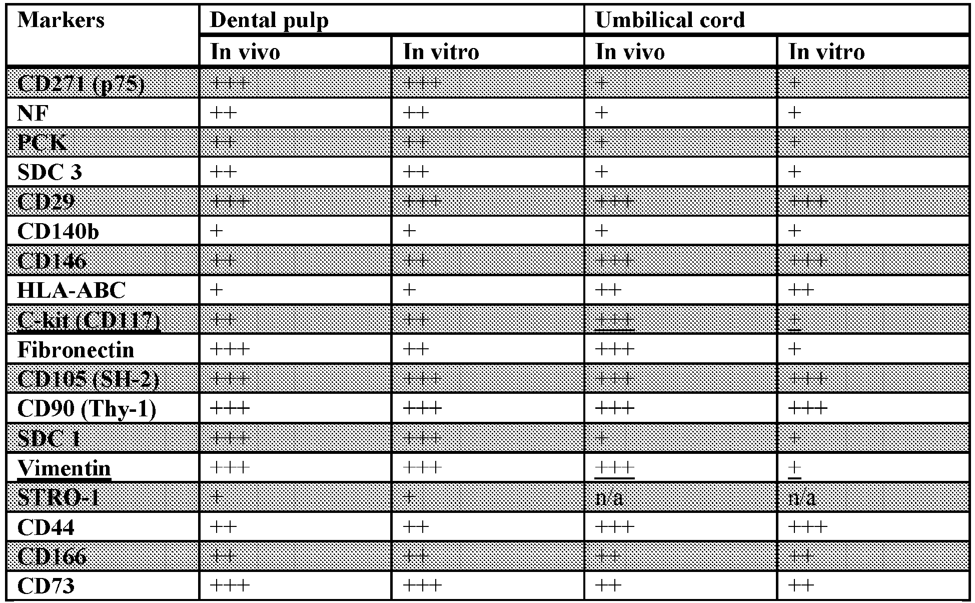

Unless defined otherwise, all technical and scientific terms herein have the same meaning as commonly understood by one of ordinary skill in the art to which this invention belongs. Although any methods and materials, similar or equivalent to those described herein, can be used in the practice or testing of the present invention, the preferred methods and materials are described herein. All publications, patents, and patent publications cited are incorporated by reference herein in their entirety for all purposes.

The publications discussed herein are provided solely for their disclosure prior to the filing date of the present application. Nothing herein is to be construed as an admission that the present invention is not entitled to antedate such publication by virtue of prior invention.

It is understood that the disclosed invention is not limited to the particular methodology, protocols and materials described as these can vary. It is also understood that the terminology used herein is for the purposes of describing particular embodiments only and is not intended to limit the scope of the present invention which will be limited only by the appended claims.

Those skilled in the art will recognize, or be able to ascertain using no more than routine experimentation, many equivalents to the specific embodiments of the invention described herein. Such equivalents are intended to be encompassed by the following claims.

### REFERENCES

1. Chen VC et al. (2012) "Scalable GMP compliant suspension culture system for human ES cells" Stem Cell Res. 2012 May;8(3):388-402.
2. Schirmaier et al: Scale-up of adipose tissue-derived mesenchymal stem cell production in stirred single-use bioreactors under low-serum conditions. Eng. Life Sci. 2014, 14, 292-303,
3. Vallee et al: Adipose-tissue engineering: taking advantage of the properties of human adipose-derived stem/stromal cells. Pathol Biol (Paris). 2009 Jun;57(4):309-17
4. Nekanti et al: Optimization and scale-up of Wharton's jelly-derived mesenchymal stem cells for clinical applications. Stem Cell Research (2010) 5, 244-254.
5. Brooke G, Rossetti T, Pelekanos R, et al: Manufacturing of human placenta-derived mesenchymal stem cells for clinical trials. Br J Haematol. 144:571―579. 2008.
6. Govindasamy et al: Human platelet lysate permits scale-up of dental pulp stromal cells for clinical applications. Cytotherapy, 2011; Early Online, 1-13
7. Hanley et al: Efficient manufacturing of therapeutic mesenchymal stromal cells with the use of the Quantum Cell Expansion System ;Cytotherapy, 2014-08-01, Volume 16, Issue 8, 1048-1058
8. Lizier NF, Kerkis A, Gomes CM, Hebling J, Oliveira CF, Caplan AI, Kerkis I. Scaling-up of dental pulp stem cells isolated from multiple niches. PLoS One. 2012;7(6):e39885; Kerkis I, Caplan AI. Stem cells in dental pulp of deciduous teeth. Tissue Eng Part B Rev. 2012 Apr;18(2):129-38.
9. Maher Al-Atari Abou-Asi Núria Casals Farré Lluis Giner Tarrida Lluis Giner Tarrida Eduardo Ferrés Padró Patent application title: PLURIPOTENT STEM CELLS OBTAINED FROM DENTAL PULP http://www.faqs.org/patents/app/20110236356
10. Cordeiro MM, Dong Z, Kaneko T, Zhang Z, Miyazawa M, Shi S, Smith AJ, Nor JE. Dental pulp tissue engineering with stem cells from exfoliated deciduous teeth. J Endod. 2008 34(8): 962-9.
11. Arora V, Arora P, Munshi AK. Banking Stem Cells from Human Exfoliated Deciduous Teeth (SHED): Saving for the Future J Clin Pediatr Dent 33(4): 289-294, 2009
12. Sukoyan MA, Kerkis AY, Mello MR, Kerkis IE, Visintin JA, Pereira LV. Establishment of new murine embryonic stem cell lines for the generation of mouse models of human genetic diseases. Braz J Med Biol Res. 2002 May;35(5):535-42.
13. Carta L, Pereira L, Arteaga-Solis E, Lee-Arteaga SY, Lenart B, Starcher B, Merkel CA, Sukoyan M, Kerkis A, Hazeki N, Keene DR, Sakai LY, Ramirez F.Fibrillins 1 and 2 perform partially overlapping functions during aortic development. J Biol Chem. 2006 Mar 24;281(12):8016-23
14. Kerkis A, Fonseca SA, Serafim RC, Lavagnolli TM, Abdelmassih S, Abdelmassih R, Kerkis I. In vitro differentiation of male mouse embryonic stem cells into both presumptive sperm cells and oocytes. Cloning Stem Cells. 2007 Winter;9(4):535-48.
15. Lavagnolli TM, Fonseca SA, Serafim RC, Pereira VS, Santos EJ, Abdelmassih S, Kerkis A, Kerkis I. Presumptive germ cells derived from mouse pluripotent somatic cell hybrids. Differentiation. 2009 Sep-Oct;78(2-3):124-30
16. Hayashi MA, Guerreiro JR, Cassola AC, Lizier NF, Kerkis A, Camargo AC, Kerkis I. Long-term culture of mouse embryonic stem cell-derived adherent neurospheres and functional neurons. Tissue Eng Part C Methods. 2010 Dec;16(6):1493-502.
17. Lima BL, Santos EJ, Fernandes GR, Merkel C, Mello MR, Gomes JP, Soukoyan M, Kerkis A, Massironi SM, Visintin JA, Pereira LV A new mouse model for marfan syndrome presents phenotypic variability associated with the genetic background and overall levels of Fbn1 expression. PLoS One. 2010 Nov 30;5(11):e14136.
18. Beltrão-Braga PC, Pignatari GC, Maiorka PC, Oliveira NA, Lizier NF, Wenceslau CV, Miglino MA, Muotri AR, Kerkis I. Feeder-free derivation of induced pluripotent stem cells from human immature dental pulp stem cells. Cell Transplant. 2011;20(11-12):1707-19.
19. Deepa Ponnaiyan -- Do dental stem cells depict distinct characteristics? — Establishing their "phenotypic fingerprint" Dent Res J (Isfahan). 2014 Mar-Apr; 11(2): 163-172
20. Brito et al., Imbalance of p75NTR/TrkB protein expression in Huntington's disease: implication for neuroprotective therapies. Cell Death and Disease (2013) 4, e595; doi:10.138/cddis.2013.116
21. Crisostomo PR, Wang M, Wairiuko GM, et al. High passage number of stem cells adversely affects stem cell activation and myocardial protection. Shock. 2006;26:575-80.
22. Digirolamo CM, Stokes D, Colter D, et al. Propagation and senescence of human marrow stromal cells in culture: a simple colony-forming assay identifies samples with the greatest potential to propagate and differentiate. Br J Haematol. 1999;107:275-81.
23. Muraglia A, Cancedda R, Quarto R. Clonal mesenchymal progenitors from human bone marrow differentiate in vitro according to a hierarchical model. J Cell Sci. 2000;113:1161-6.
24. Rubio D, Garcia-Castro J, Martin MC, et al. Spontaneous human adult stem cell transformation. Cancer Res. 2005;65:3035-9.
25. Stenderup K, Justesen J, Clausen C, Kassem M. Aging is associated with decreased maximal life span and accelerated senescence of bone marrow stromal cells. Bone. 2003;33:919-26.

## Claims

1. A method of expanding a primary derived stem cell population in vitro from an explant tissue, the method comprising
culturing the explant tissue in a culture medium for at least three harvest cycles, wherein a harvest cycle comprises:
maintaining the explant tissue in culture medium under nutrient deprivation and hypoxic conditions for a period of time sufficient to obtain a semi-confluent stem cell culture and collecting the semi-confluent stem cells for the final stem cell population, and wherein:
the explant tissue is mechanically transferred to a new culture medium prior to each harvest cycle;
the explant tissue is not enzymatically or chemically treated or dissected prior to each harvest cycle; and
the explant tissue is umbilical cord tissue or dental pulp.

2. The method of claim 1, wherein maintaining the explant tissue in culture medium under nutrient deprivation and hypoxic conditions comprises maintaining the explant tissue in culture medium containing less than 5% serum.

3. An in vitro method of obtaining from an explant tissue an expanded population of primary derived stem cells, the method comprising:
a. washing and minimally manipulating the explant tissue;
b. culturing the explant tissue in vitro in a first culture surface under nutrient deprivation and hypoxic conditions to establish a culture of primary derived stem cells;
c. collecting primary derived stem cells; and
d. mechanically transferring the explant tissue to second culture surface with a culture medium containing less than 5% serum;
e. repeating steps b., c., and d., thereby obtaining an expanded population of primary derived stem cells from the explant tissue;
wherein:
a harvest cycle comprises steps b. and c. and the method comprises at least three harvest cycles;
the explant tissue is not enzymatically or chemically treated or dissected prior to each harvest cycle; and
the explant tissue is umbilical cord tissue or dental pulp.

4. The method of any one of the preceding claims, further comprising the step of passaging the primary derived stem cells on a growth medium, and the passaged primary derived stem cells are collected.

5. The method of any one of the preceding claims, wherein the culture medium and the growth medium is DMEM.

6. The method of claim 5, wherein the culture medium is DMEM-F12.

7. The method of claim 4, wherein the growth medium is DMEM-low glucose.

8. The method of claim 7, wherein the growth medium is not supplemented with nonessential amino acids.

9. The method of claim 4, wherein the primary derived and passaged stem cells are mixed to form a batch of primary derived and passaged stem cells from a single explant tissue.

10. The method of claim 3, wherein the step e. is repeated whereby the method comprises at least 30 harvest cycles.

11. The method of any one of the preceding claims, wherein the explant tissue is umbilical cord tissue.

12. The method of any one of the preceding claims, wherein the explant tissue is dental pulp.

13. A therapeutic composition of stem cells comprising stem cells obtained from the method of any of claims 1 to 12.

14. The therapeutic composition of claim 13, wherein the composition comprises explanted cells obtained from the first three harvest cycles.

15. The therapeutic composition of claim 13 or 14, further comprising passaged cells obtained from at least passage 1 (P1).
